# EUROPÄISCHE PATENTSCHRIFT

(11) **EP 3 325 053 B1**
(45) Veröffentlichungstag und Bekanntmachung des Hinweises auf die Patenterteilung: **11.08.2021**
(21) Anmeldenummer: 16728970.1
(22) Anmeldetag: 14.06.2016
(51) Int. Cl.: A61M 5/32, A61M 5/46

(54) **SICHERHEITSVORRICHTUNG FÜR EINE SPRITZE**
SAFETY DEVICE FOR A SYRINGE
DISPOSITIF DE SÉCURITÉ POUR UNE SERINGUE

(30) Priorität: 21.07.2015 DE 102015111835
(43) Veröffentlichungstag der Anmeldung: 30.05.2018
(73) Patentinhaber: Gerresheimer Regensburg GmbH, 93047 Regensburg (DE)
(72) Erfinder: FRAAS, Andreas, 92224 Amberg (DE); VOGL, Maximilian, 92708 Mantel (DE)
(74) Vertreter: Hannke, Christian
(86) Internationale Anmeldenummer: PCT/EP2016/063651
(87) Internationale Veröffentlichungsnummer: WO 2017/012783

(56) Entgegenhaltungen:
- WO-A1-2009/137845
- WO-A2-2004/032989
- US-A1- 2012 265 149
- US-A1- 2014 378 910
- US-A1- 2015 018 773

## Beschreibung

Die Erfindung betrifft eine Sicherheitsvorrichtung zur Vermeidung von Stichverletzungen für eine Spritze mit einem Spritzenkörper und einem an dem distalen Ende des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt, wobei das Hülsenelement an seinem distalen Ende einen bei einer Anwendung der Spritze zumindest abschnittsweise die Haut eines Patienten kontaktierenden Kontaktabschnitt umfasst, welcher eine Öffnungsfläche begrenzt.

Gattungsgemäße Sicherheitsvorrichtungen zur Vermeidung von Stichverletzungen sind aus dem Stand der Technik bekannt, siehe zum Beispiel WO 2009/137845. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzen der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist.

Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung ist beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können.

Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Derartige gattungsgemäße Sicherheitshülsen sind im Wesentlichen rotationssymmetrisch ausgestaltet. Demzufolge erlauben diese Sicherheitshülsen lediglich eine senkrechte Injektion des Spritzeninhaltes. Neben dieser 90°-Injektion gibt es jedoch Einsatzgebiete, welche eine 45°-Injektion erfordern. Ein solche 45°-Injektion ist beispielsweise erforderlich bei Patienten mit besonders wenig Fettschicht oder wenn Wirkstoffe in andere Schichten des Körpers injiziert werden sollen. Mit den bestehenden Sicherheitseinrichtungen ist eine derartige abgewinkelte Injektion in einem bestimmten, von 90° abweichenden Winkel nur schwer beziehungsweise unter Umgehung der Sicherheitsfunktion möglich. So wird zum Beispiel die Sicherheitshülse bereits vor der Injektion unsachgemäß zurückgezogen und festgehalten. Ein solche unsachgemäße Verwendung kann ein erhöhtes Risiko von Nadelstichverletzungen zur Folge haben. Ferner gibt es im Stand der Technik Sicherheitseinrichtungen, welche im Ausgangszustand bereits eine stark hervorstehende Nadel aufweisen, um derartige von 90° abweichende Injektionen zu ermöglichen. Eine solche Lösung wird beispielsweise in der WO 2013/ 134465 A1 offenbart. Jedoch sind solche Lösungen von Haus aus als unsicher anzusehen, da die Sicherheitsfunktion der Sicherheitseinrichtung beeinträchtigt ist. Zudem ist das Verfahren der Sicherheitshülse nach erfolgter Injektion unangenehm für den Patienten, da die Sicherheitshülse sehr punktuell mit einer Kante auf die Haut drückt.

Aufgabe der vorliegenden Erfindung ist es demnach, eine Sicherheitsvorrichtung zur Vermeidung von Stichverletzungen für eine Spritze zur Verfügung zu stellen, welche die eingangs genannten Probleme löst.

Diese Aufgabe wird gelöst durch eine Vorrichtung gemäß Anspruch 1 mit einer Sicherheitsvorrichtung zur Vermeidung von Stichverletzungen für eine Spritze mit einem Spritzenkörper und einem an dem distalen Ende des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt, wobei das Hülsenelement an seinem distalen Ende einen bei einer Anwendung der Spritze zumindest abschnittsweise die Haut eines Patienten kontaktierenden Kontaktabschnitt umfasst, welcher eine Öffnungsfläche begrenzt. Die Sicherheitsvorrichtung zeichnet sich dadurch aus, dass die Öffnungsfläche zumindest abschnittsweise einen Teilbereich aufweist, der einen Winkel α mit der axialen Richtung (X) einschließt, wobei der Winkel α in einem Bereich zwischen 20° und 70° liegt.

Durch eine derartige Ausgestaltung der Sicherheitsvorrichtung ist eine Injektion in einem von 90° abweichenden Winkel ermöglicht und gleichzeitig ist die Sicherheitsfunktion der Sicherheitsvorrichtung vor, während und nach der Injektion gewährleistet. Die Öffnungsfläche, durch welche das Stechmittel bei der Anwendung der Spritze hindurch tritt, ist demnach in einem Winkel α zu der axialen Richtung (X) beziehungsweise zu einer fiktiven Ebene, welche rechtwinklig zu der axialen Richtung liegt, orientiert. Bei einer Injektion würde diese fiktive Ebene der Ansatzfläche auf der Haut entsprechen. Der Winkel α kann je nach Bedarf ausgelegt werden. Eine Injektion in einem bestimmten Winkel erfolgt bisher mehr oder weniger nach Gefühl und ist somit stark abhängig vom Benutzer. Die erfindungsgemäße Sicherheitsvorrichtung würde hier als eine Art Lehre die Qualität (Einhaltung des Winkels a) des erzeugten Einstiches maßgeblich verbessern.

Vorteilhafterweise ist durch eine Kombination der Auslegung des Winkels α mit der Auslegung einer Länge des Stechmittels beziehungsweise des maximalen Austrittes des Stechmittels aus der Sicherheitsvorrichtung eine Einstichtiefe des Stechmittels sehr genau festlegbar. Demnach können individuelle Spritzen bereitgestellt werden, durch welche eine Injektion in einen bestimmten Körperbereich mit einer bestimmten Einstichtiefe ermöglicht wird. Vorteilhafterweise ist durch eine Kombination der Auslegung des Winkels α mit der Auslegung einer Länge des Stechmittels und/oder eines maximalen Hervortretens des Stechmittels aus der Sicherheitsvorrichtung eine Einstichtiefe des Stechmittels in die Haut eines Patienten festlegbar. Unter dem maximalen Hervortreten des Stechmittels aus der Sicherheitsvorrichtung ist der Anteil der Länge des Stechmittels zu verstehen, welcher maximal über die Öffnungsfläche hinausragen kann. Demnach können individuelle Spritzen bereitgestellt werden, durch welche eine Injektion in einen bestimmten Körperbereich mit einer bestimmten Einstichtiefe ermöglicht wird.

Besonders bevorzugt liegt der Winkel α bei 45°. Insbesondere Injektionen mit einem Winkel von 45° finden häufig in der Medizin Anwendung.

Die Sicherheitsvorrichtung umfasst ein Kragenelement, welches an dem distalen Endbereich des Spritzenkörpers anordenbar ist und die Sicherheitsvorrichtung an dem Spritzenkörper entlang der axialen Richtung arretiert. Um Manipulation oder Fehlbenutzung der Spritze vorzubeugen, muss die Sicherheitsvorrichtung nicht oder nur schwer von der Spritze mit einer Kanüle entfernbar sein. Demnach ist ein entsprechend fester Sitz in axialer Richtung notwendig.

Das Kragenelement weist zumindest einen Führungsvorsprung auf, welcher in zumindest einer Führungskulisse des Hülsenelements eingreift, wobei der Führungsvorsprung in der zumindest einen Führungskulisse des Hülsenelements bei einer Relativbewegung des Spritzenkörpers zu dem Hülsenelement im Wesentlichen entlang der axialen Richtung (X) geführt ist. Das Kragenelement ist in einer Umfangsrichtung (U) rotierbar an dem distalen Endbereich des Spritzenkörpers angeordnet. Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse wird eine Rotation des Kragenelements entlang einer Umfangsrichtung (U) verursacht. Das Hülsenelement schiebt sich dadurch vorzugsweise über den Spritzenkörper, wodurch das Stechmittel, welches eine Kanüle, eine Nadel oder auch eine Lanzette sein kann, durch eine entsprechende Öffnung in dem Hülsenelement hindurchtritt. Somit wird eine Rotation des Hülsenelements auf der Haut des Patienten um die Einstichstelle herum vermieden.

Gemäß einer besonders bevorzugten Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird.

Gemäß einer bevorzugten Ausführungsform umfasst das Kragenelement einen distalen Bereich, in welchem die Wandung des Kragenelements zumindest zwei Schlitze aufweist, welche sich in axialer Richtung (X) erstrecken. Durch derartige Schlitze ist eine Anpassung des Kragenelements an unterschiedliche Spritzenkörperformen beziehungsweise Spritzenkörperdurchmesser gewährleistet. Ferner ist durch die Schlitze das Aufbringen des Kragenelements auf den Spritzenkörper erleichtert. Beim Aufbringen des Kragenelements auf den Spritzenkörper wird üblicherweise das Kragenelement auf den Spritzenkörper geschoben. Weist nun der Spritzenkörper einen Vorsprung oder eine Verdickung auf, welche beispielsweise zur Arretierung des Kragenelements in axialer Richtung dienen kann, kann ein Aufbringen erschwert werden. Durch die vorteilhaften Schlitze ist jedoch eine geringe Aufweitung des Kragenelements möglich, wodurch dieses leichter auf den Spritzenkörper aufgeschoben werden kann.

Vorzugsweise ist der Spritzenkörper als hohler Kreiszylinder ausgestaltet und weist in seinem distalen Endbereich ein konisches Endstück auf, an welchem das Stechmittel angeordnet ist. Vorzugsweise besteht der Spritzenkörper aus Glas oder aus einem Polymer-Kunststoff, bevorzugt einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC). Weiter bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar ist. Weiterhin bevorzugt ist auch die Sicherheitsvorrichtung im Wesentlichen in Form eines hohlen Kreiszylinders ausgestaltet.

Nach einem weiteren bevorzugten Gedanken der Erfindung umfasst der Kontaktabschnitt eine Stirnfläche. Diese Stirnfläche liegt bei Anwendung der Spritze auf der Haut des Patienten auf. Demnach ist es vorteilhaft, die Reibung zwischen der Stirnfläche und der Haut zu erhöhen, um ein Abrutschen der Spritze zu unterbinden. Demnach ist es vorteilhaft, dass diese eine angeraute Oberflächenstruktur aufweist und/oder aus einem gummierten Material besteht. Die Stirnfläche kann beispielsweise angeraut geformt sein oder eine Beschichtung aus einem angerauten Material aufweisen.

Gemäß einer weiteren Ausführungsform weist die Öffnungsfläche einen weiteren Teilbereich auf, welcher einen Winkel β mit der axialen Richtung (X) einschließt, wobei der Winkel β in einem Bereich zwischen 80° und 100° liegt. Bevorzugt beträgt der Winkel β 90°.

Nach einem weiteren vorteilhaften Gedanken der Erfindung umfasst die zumindest eine Führungskulisse einen ersten und einen zweiten Kulissenbereich, die durch eine entlang der axialen Richtung (X) des Spritzenkörpers verlaufende fiktive Trennlinie voneinander getrennt sind, wobei der Führungsvorsprung in einer Ausgangsstellung in dem ersten Kulissenbereich anordenbar ist.

Bevorzugt ist in der Ausgangsstellung ein distales Ende des Stechmittels in axialer Richtung (X) innerhalb des Hülsenelements angeordnet. Die Öffnungsfläche des Hülsenelements ist also in axialer Richtung (X) über dem distalen Ende des Stechmittels angeordnet. Vorzugsweise ist der Führungsvorsprung von dem ersten in den zweiten Kulissenbereich in eine Endstellung durch Überschreiten der Trennlinie überführbar, wenn ein distales Ende des Stechmittels bei der Relativbewegung von dem Spritzenkörper zu dem Hülsenelement in der Öffnungsfläche angeordnet ist. Demnach ist der Führungsvorsprung von dem ersten Kulissenbereich in den zweiten Kulissenbereich überführbar. Diese Überführung findet statt, wenn der Führungsvorsprung eine fiktive Trennlinie, welche den ersten und den zweiten Kulissenbereich voneinander trennt, überschreitet. Befindet sich der Führungsvorsprung in dem ersten Kulissenbereich, also in einer Ausgangsstellung, so wurde die Spritze noch nicht ausgelöst, d.h. das Stechmittel hat die Sicherheitsvorrichtung noch nicht verlassen. Befindet sich der Führungsvorsprung in dem zweiten Kulissenbereich, so ist das Stechmittel aus der Sicherheitsvorrichtung bereits ausgetreten, so dass eine Injektion möglich ist. Beim Übergang von dem ersten Kulissenbereich zu dem zweiten Kulissenbereich, also genau dann, wenn der Führungsvorsprung die Trennlinie überschreitet, befindet sich das distale Ende des Stechmittels in der Öffnungsfläche.

Gemäß einer weiteren Ausführungsform ist in der Ausgangsstellung ein distales Ende des Stechmittels in der Öffnungsfläche angeordnet.

Denkbar wäre auch, dass in der Ausgangsstellung ein distales Ende des Stechmittels in axialer Richtung (X) über das Hülsenelement beziehungsweise die Öffnungsfläche hinausragt. Demnach ist ein distales Ende des Stechmittels in axialer Richtung oberhalb der Öffnungsfläche angeordnet. Derartige Ausführungsformen wären denkbar, wenn eine entsprechend große Einstichtiefe erzielt werden soll.

Vorzugsweise weist die Sicherheitsvorrichtung zumindest ein Federelement auf, das mit dem Spritzenkörper wirkverbunden ist und der Relativbewegung des Spritzenkörpers zu der Sicherheitsvorrichtung entgegenwirkt. Demnach bleibt das Stechmittel bis zur vorgesehenen Anwendung innerhalb des Hülsenelements. Bei der Anwendung muss das Hülsenelement gegen die Federkraft verschoben werden, damit das Stechmittel durch die Öffnung des Hülsenelements hindurchtreten beziehungsweise in eine Injektionsposition verschoben werden kann. Nach Gebrauch der Spritze schiebt sich automatisch, angetrieben durch die Federkraft des Federelements, das Hülsenelement wieder zumindest teilweise über das Stechmittel. Durch die Führung des Führungsvorsprungs in der Führungskulisse rotiert das Kragenelement entgegen der Umfangsrichtung (U). Der Anwender ist somit vor Stichverletzungen mit dem gebrauchten kontaminierten Stechmittel geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Bevorzugt ist der Führungsvorsprung von dem zweiten Kulissenbereich mittels einer Kulisse des zweiten Kulissenbereichs in einen Endbereich überführbar, in welchem eine Relativbewegung des Hülsenelements zu dem Spritzenkörper im Wesentlichen entlang der axialen Richtung (X) zumindest eingeschränkt ist. Durch eine derartige Ausgestaltung ist ein weiteres Verschieben des Hülsenelements relativ zum Spritzenkörper zumindest eingeschränkt, bevorzugt verhindert. Demzufolge ist ein weiteres Austreten des Stechmittels aus der Sicherheitsvorrichtung nach der Anwendung der Spritze unterbunden.

Nach einem weiteren bevorzugten Gedanken umfasst die Sicherheitseinrichtung ein Kappenelement, welches an dem distalen Ende des Hülsenelements angeordnet ist. Es wäre denkbar, dass durch ein solches Kappenelement das Hülsenelement bezüglich der Relativbewegung des Spritzenkörpers zu dem Hülsenelement arretierbar ist. Demnach wird effektiv ein unerwünschtes Hervortreten des Stechmittels, welches eine Kanüle, eine Nadel oder auch eine Lanzette sein kann, aus der Sicherheitsvorrichtung unterbunden. Beschädigungen und Kontamination des Stechmittels werden demnach verhindert. Der Anwender der Spritze muss zunächst das Kappenelement von dem Hülsenelement abziehen, bevor die Spritze angewendet werden kann. Demnach wird ebenso das Risiko eines versehentlichen Betätigens verringert. Es wäre denkbar, an dem Kappenelement eine Markierung oder einen Hinweis anzubringen. Der Anwender wäre somit gezwungen, diese Markierung beziehungsweise den Hinweis vor der Anwendung der Spritze wahrzunehmen. Eine derartige Markierung beziehungsweise ein derartiger Hinweis könnte farblich und/oder haptisch und/oder anderweitig ausgestaltet sein. Insbesondere für Ausführungsformen der Sicherheitsvorrichtung, bei denen das distale Ende des Stechmittels bereits in der Ausgangsposition über die Sicherheitsvorrichtung hinausragt, ist ein Kappenelement sinnvoll, um das Stechmittel vor Beschädigungen und den Anwender vor Verletzungen zu schützen. Es ist weiterhin denkbar, dass das Kappenelement eine Stechmittelschutzeinrichtung aufweist. Eine solche Stechmittelschutzeinrichtung könnte beispielsweise aus einem elastischen Material, beispielsweise Gummi hergestellt sein. Ein solches elastische Material begünstigt eine Reduzierung des Beschädigungsrisikos des Stechmittels. Vorstellbar wäre auch, dass das Kappenelement eine Aufsatzfläche aufweist, welche an dem Kontaktabschnitt anliegt und somit komplementär zu diesem ausgebildet wäre. Ein abschließendes distales Ende des Kappenelements könnte dann rechtwinklig zu der axialen Richtung (X) ausgestaltet werden. Es ist denkbar, dass das Kappenelement beziehungsweise das Hülsenelement Rastelemente aufweist, durch welche das Kappenelement an dem Hülsenelement befestigt werden kann. Vorstellbar wären auch anderweitige Befestigungsarten, wie beispielsweise das Vorsehen ineinanderoder umgreifender Elemente an dem Kappenelement und/oder dem Hülsenelement.

Vorzugsweise umfasst das Stechmittel einen Schliff und eine Spritze. Vorteilhafterweise ist das Stechmittel derart angeordnet, dass dessen Spitze in einer zu der maximalen Höhe des Hülsenelements diametral gegenüberliegenden Richtung angeordnet ist. Durch eine solche Ausrichtung des Stechmittels bezüglich des Hülsenelements wird eine optimale Einstichposition gewährleistet, wodurch dem Patienten weniger Schmerzen bei dem Einstich verursacht werden. Stechmittel, beispielsweise Kanülen, weisen einen Schliff auf. Häufig wird ein sogenannter Facettenschliff verwendet. Das distale Ende einer solchen Kanüle weist demnach zumeist zumindest einen Neigungswinkel und eine Spitze, welche außermittig an dem Rand der Kanüle angeordnet ist, auf. Wird eine Injektion unter einem bestimmten Winkel durchgeführt, kann eine ungünstige Positionierung der Spitze der Kanüle bezüglich des Hülsenelements dem Patienten unnötige Schmerzen verursachen.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: eine isometrische Ansicht einer Spritze mit Sicherheitsvorrichtung;
- Fig.2: eine Seitenansicht einer Spritze mit Sicherheitsvorrichtung;
- Fig.3: eine isometrische Ansicht einer Spritze mit Sicherheitsvorrichtung gemäß einer weiteren Ausführungsform;
- Fig.4: eine Seitenansicht einer Spritze mit Sicherheitsvorrichtung gemäß einer weiteren Ausführungsform;
- Fig.5: eine isometrische Ansicht einer Spritze mit Sicherheitsvorrichtung gemäß einer weiteren Ausführungsform;
- Fig. 6: eine isometrische Ansicht einer Spritze mit Sicherheitsvorrichtung gemäß einer weiteren Ausführungsform.

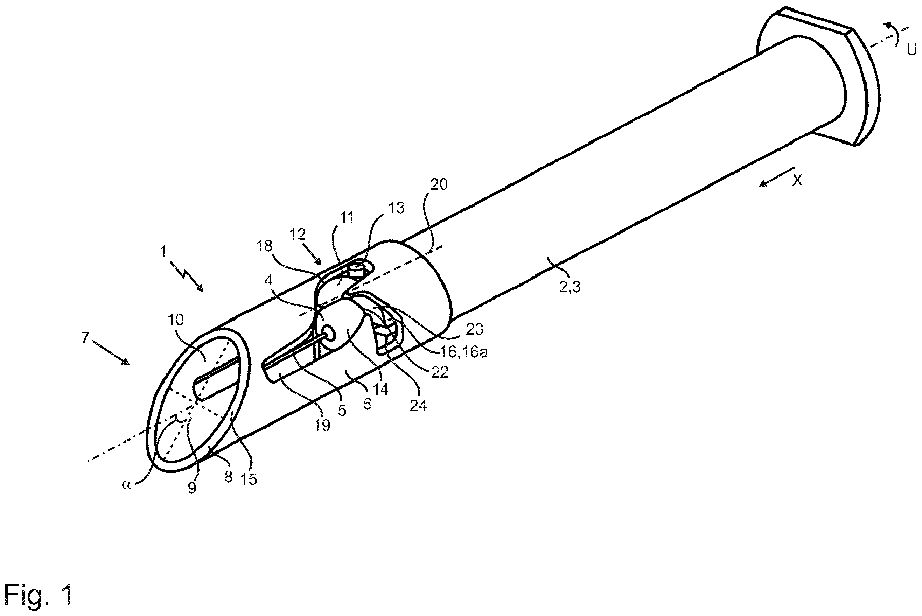

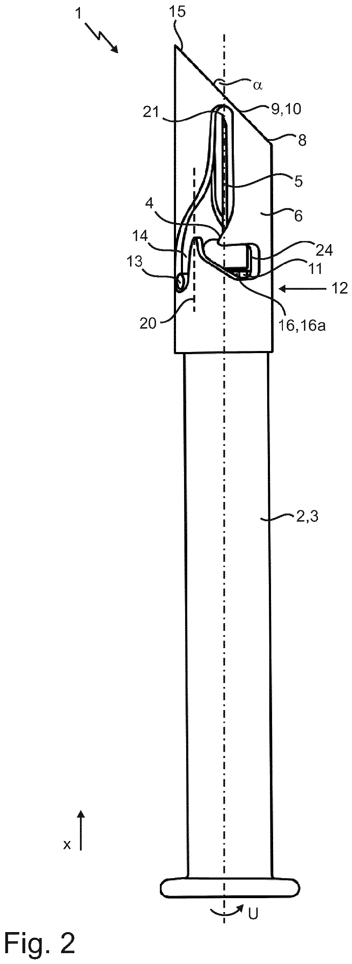

In Fig. 1 ist eine isometrische eine isometrische Ansicht einer Spritze mit Sicherheitsvorrichtung und in Fig. 2 ist eine Seitenansicht einer Spritze mit Sicherheitsvorrichtung gemäß einer ersten Ausführungsform dargestellt.

Die Spritze (2) umfasst einen Spritzenkörper (3), welcher als hohler Kreiszylinder ausgestaltet ist. Der Spritzenkörper weist einen distalen Endbereich (12) mit einem distalen Ende (4) auf. An dem distalen Ende (4) ist ein Stechmittel (5) angeordnet. Dieses Stechmittel (5) ist über eine Bohrung in dem distalen Endbereich (12) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (12) ist als konisches Endstück ausgestaltet, welches einen kleineren Außendurchmesser als der Spritzenkörper (3) aufweist. Weiterhin weist die Spritze einen Übergangsbereich auf, in dem der Außendurchmesser des Spritzenkörpers (3) in den Außendurchmesser des Endstücks übergeht.

Die Sicherheitsvorrichtung (1) zur Vermeidung von Stichverletzungen für eine Spritze (2) mit einem Spritzenkörper (3) und einem an dem distalen Ende (4) des Spritzenkörpers (3) angeordneten Stechmittel (5) umfasst ein sich entlang einer axialen Richtung (X) erstreckendes Hülsenelement (6), welches zumindest teilweise das Stechmittel (6) und den Spritzenkörper (3) umschließt. Das Hülsenelement (6) umfasst an seinem distalen Ende (7) einen bei einer Anwendung der Spritze (2) zumindest abschnittsweise die Haut eines Patienten kontaktierenden Kontaktabschnitt (8), welcher eine Öffnungsfläche (9) begrenzt. Die Öffnungsfläche (9) weist zumindest abschnittsweise einen Teilbereich (10) auf, der einen Winkel α mit der axialen Richtung (X) einschließt, wobei der Winkel α in einem Bereich zwischen 20° und 70° liegt.

In dieser Ausführungsform weist die Öffnungsfläche lediglich einen Teilbereich (10) auf und der Winkel α beträgt 45°. Die Sicherheitsvorrichtung (1) ist im Wesentlichen als hohler Kreiszylinder ausgebildet. Demzufolge weist die Öffnungsfläche (9) eine elliptische Form auf. Durch eine derartige Ausgestaltung der Sicherheitsvorrichtung (1) ist eine Injektion in einem von 90° abweichenden Winkel ermöglicht und gleichzeitig ist die Sicherheitsfunktion der Sicherheitsvorrichtung (1) vor, während und nach der Injektion gewährleistet. Die Öffnungsfläche, durch welche das Stechmittel bei der Anwendung der Spritze hindurch tritt, ist demnach in einem Winkel von 45° zu der axialen Richtung (X) beziehungsweise zu einer fiktiven Ebene, welche rechtwinklig zu der axialen Richtung (X) liegt, orientiert. Bei einer Injektion würde diese fiktive Ebene der Ansatzfläche auf der Haut entsprechen. Eine Injektion in einem bestimmten Winkel erfolgt bisher mehr oder wenig nach Gefühl und ist somit stark abhängig vom Benutzer. Die Sicherheitsvorrichtung (1) würde hier als eine Art Lehre die Qualität (Einhaltung der 45°) des erzeugten Einstiches maßgeblich verbessern. Der die Öffnungsfläche (9) begrenzende Kontaktabschnitt (8) umfasst eine Stirnfläche (15). Diese Stirnfläche (15) kontaktiert bei einer Anwendung der Spritze (2) die Haut des Patienten. Um ein Abrutschen der Spritze zu vermeiden, kann diese Stirnfläche (15) eine angeraute Oberflächenstruktur aufweisen und/oder aus einem gummierten Material bestehen.

Die Sicherheitsvorrichtung (1) umfasst weiterhin ein Kragenelement (11), welches an dem distalen Endbereich (12) des Spritzenkörpers (3) anordenbar ist. Die Sicherheitsvorrichtung (1) wird durch das Kragenelement (11) an dem Spritzenkörper (3) bezüglich der axialen Richtung (X) arretiert. Das Kragenelement (10) ist weiter im Wesentlichen als hohler Kreiszylinder (16) ausgebildet. Die Arretierung in axialer Richtung wird durch einen Vorsprung (22) bzw. eine Verdickung an dem distalen Ende (4) des Spritzenkörpers (3) ermöglicht, an welchem das Kragenelement (11) mit seinem distalen Ende (23) anliegt. Ferner ist das Kragenelement (11) in einer Umfangsrichtung (U) rotierbar an dem distalen Endbereich (12) des Spritzenkörpers (3) angeordnet. Ferner weist der Kreiszylinder (16) eine Mantelfläche (16a) auf, an der zwei Führungsvorsprünge (13) angeordnet sind. Die Führungsvorsprünge (13) erstrecken sich radial von der Mantelfläche (16a) nach außen weg und sind diametral gegenüberliegend angeordnet. Weiterhin sind diese als Kreiszylinder beziehungsweise als Stift ausgebildet. Diese beiden Führungsvorsprünge (13) greifen in jeweils eine Führungskulisse (14) des Hülsenelements (6) ein und sind in diesen bei einer Relativbewegung des Spritzenkörpers (3) zu dem Hülsenelement (6) im Wesentlichen entlang der axialen Richtung (X) geführt. Die Sicherheitsvorrichtung (1) weist in einer Ausführungsform weiterhin ein Federelement (in den Figuren nicht gezeigt) auf, das mit dem Spritzenkörper (3) wirkverbunden ist und der Relativbewegung des Hülsenelements (6) zu der Sicherheitsvorrichtung (1) entgegenwirkt.

Die Führungskulissen (14) des Hülsenelements (6) umfassen in der gezeigten Ausführungsform einen ersten (18) und einen zweiten Kulissenbereich (19), welche durch eine entlang der axialen Richtung (X) des Spritzenkörpers (3) verlaufende fiktive Trennlinie (20) voneinander getrennt sind, wobei der Führungsvorsprung (13) in einer Ausgangsstellung in dem ersten Kulissenbereich (18) anordenbar ist und von dem ersten (18) in den zweiten Kulissenbereich (19) in eine Endstellung durch Überschreiten der Trennlinie (20) überführbar ist, wenn ein distales Ende (21) des Stechmittels (5) bei der Relativbewegung von Spritzenkörper (3) zu Hülsenelement (6) in der Öffnungsfläche (10) angeordnet ist. Das distale Ende (21) des Stechmittels (5) ist in dieser Ausführungsform in der Ausgangsstellung in axialer Richtung (X) innerhalb des Hülsenelements (6) angeordnet. Demnach ist die Öffnungsfläche (9) in axialer Richtung über dem distalen Ende (21) des Stechmittels (5) angeordnet. Ferner weist das Hülsenelement (6) einen Endbereich (22) auf. Die Führungsvorsprünge (11) sind dabei von dem zweiten Kulissenbereich (20) mittels einer Kulisse des zweiten Kulissenbereichs (20) in einen Endbereich (24) überführbar. In diesem Endbereich (24) ist eine Relativbewegung des Hülsenelements (6) zu dem Spritzenkörper (3) im Wesentlichen entlang der axialen Richtung (X) zumindest eingeschränkt.

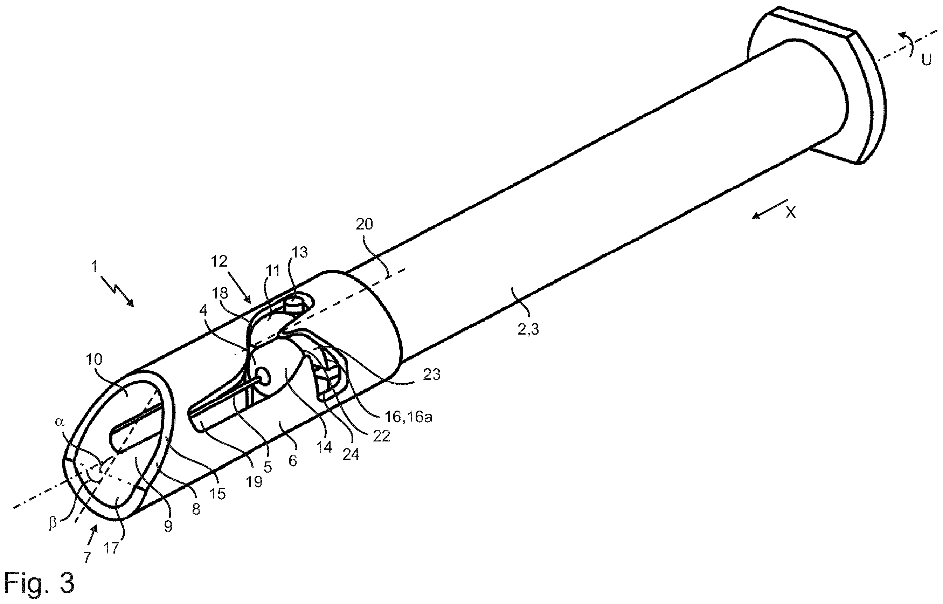

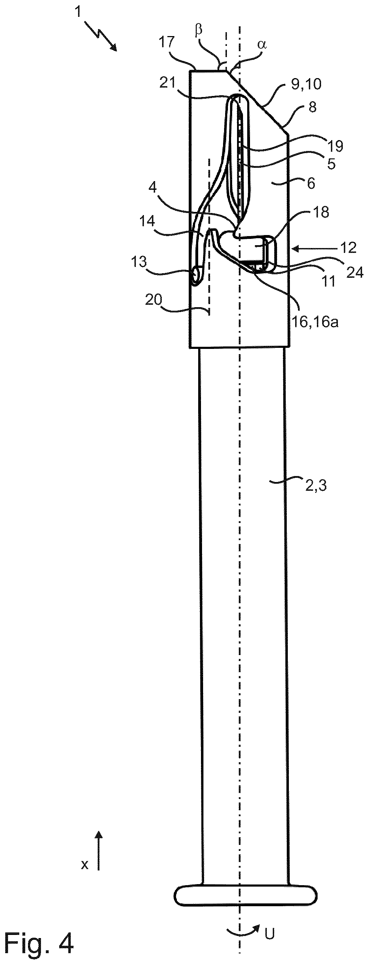

In Fig. 3 ist eine isometrische Ansicht einer Spritze mit Sicherheitsvorrichtung und in Fig. 4 eine Seitenansicht einer Spritze mit Sicherheitsvorrichtung gemäß einer weiteren Ausführungsform dargestellt. Diese Ausführungsform unterscheidet sich von der vorangegangenen Ausführungsform lediglich in der Ausgestaltung der Öffnungsfläche (9). Die Öffnungsfläche (9) weist einen ersten Teilbereich (10) auf, welcher einen Winkel α mit der axialen Richtung (X) einschließt, wobei der Winkel α in einem Bereich zwischen 20° und 70° liegt. Die Öffnungsfläche (9) weist überdies einen weiteren Teilbereich (17) auf, welcher einen Winkel β mit der axialen Richtung (X) einschließt, wobei der Winkel β in einem Bereich zwischen 80° und 100° liegt. In diesem Fall würde ein Winkel α von 45° und ein Winkel β von 90° gewählt. Ein solcher senkrecht zu der axialen Richtung (X) verlaufender Teilbereich (17) kann als Auflagefläche für eine senkrechte Injektion dienen. Es ist aber auch denkbar, dass dieser Bereich dazu dient, ein Kappenelement an dem Hülsenelement anzuordnen. Zu diesem Zweck kann dieser Teilbereich (17) Befestigungselemente, beispielsweise Rastelemente umfassen. Der erste Teilbereich (10) dient, wie in der vorangegangenen Ausführungsform, zu einer Injektion mit einem Winkel von 45°.

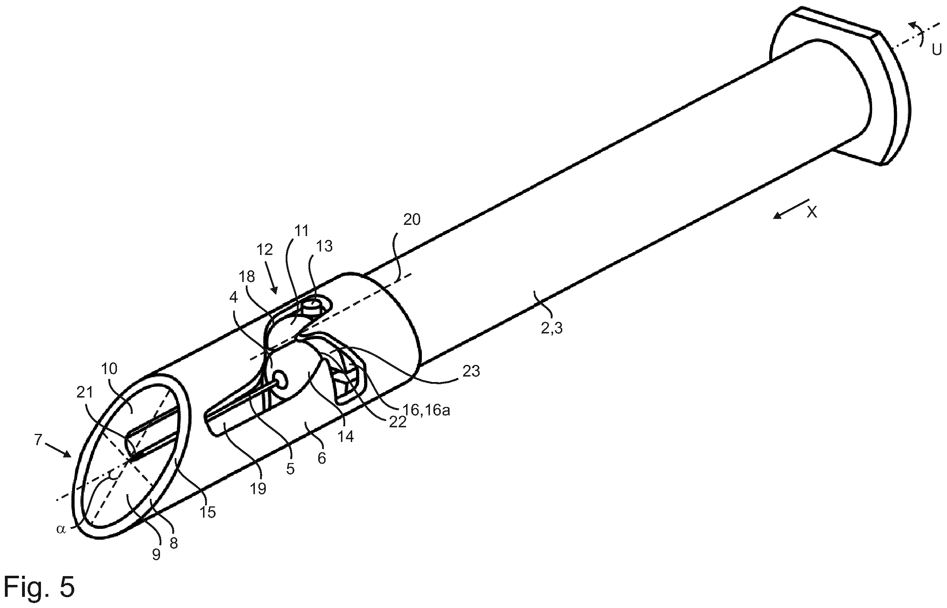

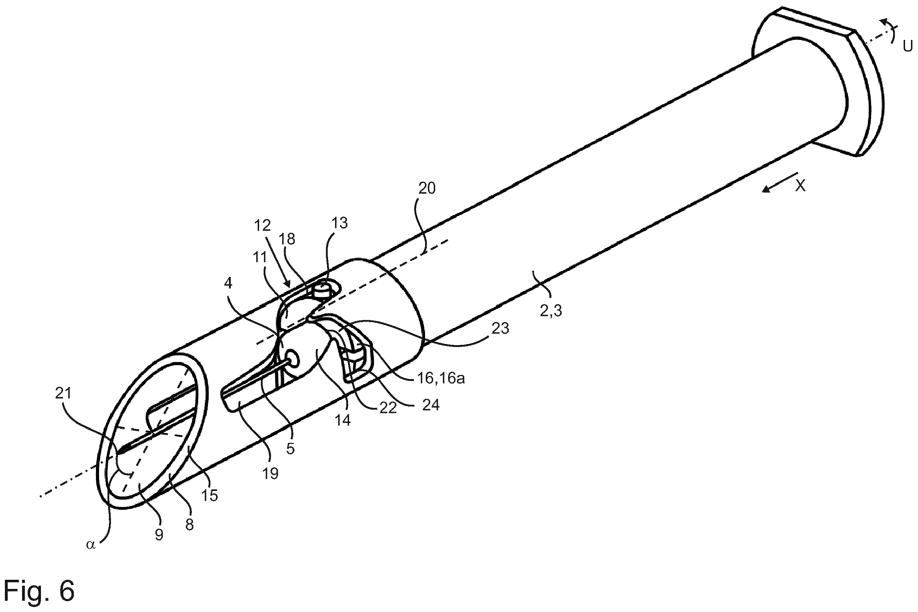

In den Figuren 5 und 6 sind Ausführungsformen der Sicherheitseinrichtung dargestellt, die im Grunde der Ausführungsform gemäß den Figuren 1 und 2 entsprechen. Sie unterscheiden sich lediglich in der Position des distalen Endes (21) des Stechmittels (5) in der Ausgangsstellung. Die Führungskulissen (14) umfassen einen ersten (18) und einen zweiten Kulissenbereich (19), die durch eine entlang der axialen Richtung (X) des Spritzenkörpers (3) verlaufende fiktive Trennlinie (20) voneinander getrennt sind, wobei die Führungsvorsprünge (13) in der Ausgangsstellung in den jeweiligen ersten Kulissenbereichen (18) anordenbar sind. In der Ausführungsform gezeigt in Fig. 1 bzw. 2 ist das distale Ende (21) des Stechmittels (5) in der Ausgangstellung in dem Hülsenelement angeordnet, d.h. die Öffnungsfläche ist in axialer Richtung (X) über dem distalen Ende (21) des Stechmittels (5) angeordnet. In Fig. 4 hingegen ist in der Ausgangsstellung das distale Ende (21) des Stechmittels (5) in der Öffnungsfläche (9) angeordnet. In Fig. 5 ist in der Ausgangsstellung das distale Ende (21) des Stechmittels (5) in axialer Richtung (X) oberhalb der Öffnungsfläche (9) angeordnet. Das distale Ende (21) des Stechmittels (5) ragt demnach in axialer Richtung (X) über das Hülsenelement hinaus. Mit derartigen Ausführungsformen kann eine entsprechend tiefe Einstichtiefe erzielt werden.

Das Stechmittel (5) bleibt bis zur vorgesehenen Anwendung der Spritze (2) innerhalb des Hülsenelements (6). Bei einer Anwendung wird die Spritze an die Haut des Patienten gepresst, wobei der Kontaktabschnitt (8) des Hülsenelements (6) bzw. dessen Stirnfläche (15) die Haut des Patienten kontaktiert. Dadurch, dass die Öffnungsfläche (9) zumindest abschnittsweise einen Teilbereich (10) aufweist, der einen Winkel α mit der axialen Richtung (X) einschließt, kann die Injektion in einem bestimmten Winkel durchgeführt werden. Das Hülsenelement (6) muss gegen die Federkraft des Federelements verschoben werden, damit das Stechmittel (5) zu der Injektionsposition verschoben werden kann. Das Hülsenelement (6) wird dabei über den distalen Endbereich (12) des Spritzenkörpers (3) geschoben. Durch die Führung des Führungsvorsprungs (13) in der Führungskulisse (14) rotiert das Kragenelement (11) entlang der Umfangsrichtung (U). Nach Gebrauch der Spritze (2) schiebt sich automatisch, angetrieben durch die Federkraft des Federelements, das Hülsenelement (6) wieder zumindest abschnittsweise über das Stechmittel (5). Durch die Führung der Führungsvorsprünge (13) in den Führungskulissen (14) rotiert das Kragenelement (11) entgegen der Umfangsrichtung (U).

### Bezugszeichenliste

- 1: Sicherheitsvorrichtung
- 2: Spritze
- 3: Spritzenkörper
- 4: distales Ende des Spritzenkörpers
- 5: Stechmittel
- 6: Hülsenelement
- 7: distales Ende des Hülsenelements
- 8: Kontaktabschnitt
- 9: Öffnungsfläche
- 10: Teilbereich der Öffnungsfläche
- 11: Kragenelement
- 12: distaler Endbereich des Spritzenkörpers
- 13: Führungsvorsprung
- 14: Führungskulisse
- 15: Stirnfläche des Kontaktabschnitts
- 16: Kreiszylinder
- 16a: Mantelfläche des Kreiszylinders
- 17: weiterer Abschnitt der Öffnungsfläche
- 18: erster Kulissenbereich
- 19: zweiter Kulissenbereich
- 20: Trennlinie
- 21: distales Ende des Stechmittels
- 22: Vorsprung des Spritzenkörpers
- 23: distalen Ende des Kragenelements
- 24: Endbereich
- X: axiale Richtung
- U: Umfangsrichtung

## Patentansprüche

1. Spritzenkörper (3) einer Spritze (2) mit einem an dessen distalen Ende (4) angeordneten Stechmittel (5), wobei an dem Spritzenkörper (3) eine Sicherheitsvorrichtung (1) zur Vermeidung von Stichverletzungen angeordnet ist, die ein sich entlang einer axialen Richtung (X) erstreckendes Hülsenelement (6) umfasst, welches zumindest teilweise das Stechmittel (5) und den Spritzenkörper (3) umschließt, wobei das Hülsenelement (6) an seinem distalen Ende (7) einen bei einer Anwendung der Spritze (2) zumindest abschnittsweise die Haut eines Patienten kontaktierenden Kontaktabschnitt (8) umfasst, welcher eine Öffnungsfläche (9) begrenzt, wobei die Öffnungsfläche (9) zumindest abschnittsweise einen Teilbereich (10) aufweist, der einen Winkel α mit der axialen Richtung (X) einschließt, wobei der Winkel α in einem Bereich zwischen 20° und 70° liegt,
wobei die Sicherheitsvorrichtung (1) ein Kragenelement (11) umfasst, welches an dem distalen Endbereich (12) des Spritzenkörpers (3) in einer Umfangsrichtung (U) rotierbar angeordnet ist und die Sicherheitsvorrichtung (1) an dem Spritzenkörper (3) entlang der axialen Richtung (X) arretiert, wobei das Kragenelement (11) zumindest einen Führungsvorsprung (13) aufweist, welcher in zumindest einer Führungskulisse (14) des Hülsenelements (6) eingreift, wobei der Führungsvorsprung (13) in der zumindest einen Führungskulisse (14) des Hülsenelements (6) bei einer Relativbewegung des Spritzenkörpers (3) zu dem Hülsenelement (6) im Wesentlichen entlang der axialen Richtung (X) geführt ist,
wobei bei einer Anwendung der Spritze (2) der zumindest eine Führungsvorsprung (13) von einem ersten Kulissenbereich (18) zu einem zweiten Kulissenbereich (19) geführt wird und ein Abschnitt des Stechmittels (5) durch die Öffnungsfläche (9) und in einem von 90°abweichenden Winkel in die Haut des Patienten tritt.

2. Spritzenkörper (3) nach Anspruch 1, wobei der Winkel α bei 45° liegt.

3. Spritzenkörper (3) nach Anspruch 1, wobei das Kragenelement (11) im Wesentlichen als hohler Kreiszylinder (16) ausgebildet ist, wobei der Kreiszylinder (16) eine Mantelfläche (16a) aufweist, an der der zumindest eine Führungsvorsprung (13) angeordnet ist.

4. Spritzenkörper (3) nach einem der vorhergehenden Ansprüche, wobei der Kontaktabschnitt (8) eine Stirnfläche (15) umfasst, welche eine angeraute Oberflächenstruktur aufweist und/oder aus einem gummierten Material besteht.

5. Spritzenkörper (3) nach einem der vorhergehenden Ansprüche, wobei die Öffnungsfläche (9) einen weiteren Teilbereich (17) aufweist, welcher einen Winkel β mit der axialen Richtung (X) einschließt, wobei der Winkel β in einem Bereich zwischen 80° und 100° liegt.

6. Spritzenkörper (3) nach einem der vorhergehenden Ansprüche, wobei die Führungskulisse (14) einen ersten (18) und einen zweiten Kulissenbereich (19) umfasst, die durch eine entlang der axialen Richtung (X) des Spritzenkörpers (3) verlaufende fiktive Trennlinie (20) voneinander getrennt sind, wobei der Führungsvorsprung (13) in einer Ausgangsstellung in dem ersten Kulissenbereich (18) anordenbar ist.

7. Spritzenkörper (3) nach Anspruch 6, wobei in der Ausgangsstellung ein distales Ende (21) des Stechmittels (5) in axialer Richtung (X) innerhalb des Hülsenelements angeordnet ist, wobei der Führungsvorsprung (13) von dem ersten (18) in den zweiten Kulissenbereich (19) in eine Endstellung durch Überschreiten der Trennlinie (20) überführbar ist, wenn ein distales Ende (21) des Stechmittels (5) bei der Relativbewegung von Spritzenkörper (3) zu Hülsenelement (6) in der Öffnungsfläche (10) angeordnet ist.

8. Spritzenkörper (3) nach Anspruch 6, wobei in der Ausgangsstellung ein distales Ende (21) des Stechmittels (5) in der Öffnungsfläche (9) angeordnet ist.

9. Spritzenkörper (3) nach Anspruch 6, wobei in der Ausgangsstellung ein distales Ende (21) des Stechmittels (5) in axialer Richtung (X) über das Hülsenelement hinausragt.

10. Spritzenkörper (3) nach einem der vorhergehenden Ansprüche, wobei die Sicherheitsvorrichtung (1) mindestens ein Federelement aufweist, das mit dem Spritzenkörper (3) wirkverbunden ist und der Relativbewegung des Spritzenkörpers (3) zu dem Hülsenelement (6) entgegenwirkt.

11. Spritzenkörper (3) nach einem der vorhergehenden Ansprüche, wobei der zumindest eine Führungsvorsprung (13) von dem zweiten Kulissenbereich (20) mittels einer Kulisse des zweiten Kulissenbereichs (19) in einen Endbereich (24) überführbar ist, in welchem eine Relativbewegung des Hülsenelements (6) zu dem Spritzenkörper (3) im Wesentlichen entlang der axialen Richtung (X) zumindest eingeschränkt ist.

12. Spritzenkörper (3) nach einem der vorhergehenden Ansprüche, wobei die Sicherheitseinrichtung (1) ein Kappenelement umfasst, welches an dem distalen Ende (7) des Hülsenelements (6) angeordnet ist.

13. Spritzenkörper (3) nach einem der vorhergehenden Ansprüche, wobei das Stechmittel (5) einen Schliff und eine Spitze umfasst, wobei das Stechmittel (5) derart angeordnet ist, dass dessen Spitze in einer zu der maximalen Höhe des Hülsenelements (6) diametral gegenüberliegenden Richtung angeordnet ist.

## Claims

1. Syringe body (3) of a syringe (2) having a piercing means (5) arranged at the distal end (4) of the syringe body (3), wherein a safety device (1) for avoiding stab wounds is arranged on the syringe body (3), the safety device comprising a sleeve element (6) which extends along an axial direction (X) and surrounds the piercing means (5) and the syringe body (3) at least in part, the sleeve element (6), at the distal end (7) thereof, having a contact portion (8) which is in contact with at least a portion of a patient's skin when using the syringe (2) and which defines an opening face (9), wherein
at least a portion of the opening face (9) comprises a segment (10) which forms an angle α together with the axial direction (X), the angle α being in a range between 20° and 70°, the safety device (1) comprising a collar element (11) which is arranged rotatably in a circumferential direction (U) on the distal end region (12) of the syringe body (3) and locks the safety device (1) on the syringe body (3) along the axial direction (X), the collar element (11) comprising at least one guide projection (13) which engages in at least one guide track (14) of the sleeve element (6), the guide projection (13) being guided in the at least one guide track (14) of the sleeve element (6) substantially in the axial direction (X) when the syringe body (3) moves relative to the sleeve element (6),
wherein, during an application of the syringe (2), the at least one guide projection (13) is guided from a first track region (18) to a second track region (19) and a portion of the piercing means (5) passes through the opening face (9) and into the patient's skin at an angle deviating from 90°.

2. Syringe body (3) according to claim 1,
wherein the angle α is 45°.

3. Syringe body (3) according to claim 1,
wherein the collar element (11) is substantially in the form of a hollow circular cylinder (16), the circular cylinder (16) having a lateral face (16a) on which the at least one guide projection (13) is arranged.

4. Syringe body (3) according to any of the preceding claims,
wherein the contact portion (8) comprises an end face (15) which has a roughened surface structure and/or consists of a rubberised material.

5. Syringe body (3) according to any of the preceding claims,
wherein the opening face (9) comprises another segment (17) which forms an angle β together with the axial direction (X), the angle β being in a range between 80° and 100°.

6. Syringe body (3) according to any of the preceding claims,
wherein the guide track (14) comprises a first (18) and a second (19) track region which are separated from one another by a fictive separating line (20) extending along the axial direction (X) of the syringe body (3), the guide projection (13), in a starting position, being arrangeable in the first track region (18).

7. Syringe body (3) according to claim 6,
wherein in the starting position, a distal end (21) of the piercing means (5) is arranged inside the sleeve element in the axial direction (X), it being possible for the guide projection (13) to be moved from the first track region (18) into an end position in the second (19) track region by crossing the separating line (20) when a distal end (21) of the piercing means (5) is arranged in the opening face (10) when the syringe body (3) moves relative to the sleeve element (6).

8. Syringe body (3) according to claim 6
wherein in the starting position, a distal end (21) of the piercing means (5) is arranged in the opening face (9).

9. Syringe body (3) according to claim 6,
wherein in the starting position, a distal end (21) of the piercing means (5) protrudes beyond the sleeve element in the axial direction (X).

10. Syringe body (3) according to any of the preceding claims,
wherein the safety device (1) comprises at least one spring element which is operatively connected to the syringe body (3) and counteracts the movement of the syringe body (3) relative to the sleeve element (6).

11. Syringe body (3) according to any of the preceding claims,
wherein the at least one guide projection (13) can be moved, by means of a track in the second track region (19), from the second track region (20) into an end region (24) in which a movement of the sleeve element (6) relative to the syringe body (3) substantially along the axial direction (X) is at least restricted.

12. Syringe body (3) according to any of the preceding claims,
wherein the safety device (1) comprises a cap element which is arranged at the distal end (7) of the sleeve element (6).

13. Syringe body (3) according to any of the preceding claims,
wherein the piercing means (5) comprises a cut and a tip, the piercing means (5) being arranged in such a way that the tip thereof is arranged in a direction which is diametrically opposed to the maximum height of the sleeve element (6).

## Revendications

1. Corps de seringue (3) d'une seringue (2) avec un moyen de piqûre (5) disposé à son extrémité distale (4), un dispositif de sécurité (1) pour éviter des blessures par piqûre étant disposé sur le corps de seringue (3), lequel dispositif de sécurité comporte un élément manchon (6) qui s'étend le long d'une direction axiale (X) et qui entoure au moins partiellement le moyen de piqûre (5) et le corps de seringue (3), l'élément manchon (6) comportant à son extrémité distale (7) une partie de contact (8) qui vient en contact avec la peau d'un patient au moins par parties lors d'une utilisation de la seringue (2) et qui délimite une face d'ouverture (9),
dans lequel la face d'ouverture (9) présente, au moins par parties, une zone partielle (10) qui forme un angle α avec la direction axiale (X), l'angle α se situant dans une plage entre 20° et 70°,
dans lequel le dispositif de sécurité (1) comporte un élément collier (11), lequel est disposé de manière rotative sur la zone d'extrémité distale (12) du corps de seringue (3) dans une direction périphérique (U) et bloque le dispositif de sécurité (1) sur le corps de seringue (3) le long de la direction axiale (X), l'élément collier (11) présentant au moins une saillie de guidage (13), laquelle s'engage dans au moins une coulisse de guidage (14) de l'élément manchon (6), la saillie de guidage (13) étant guidée dans ladite au moins une coulisse de guidage (14) de l'élément de manchon (6) essentiellement le long de la direction axiale (X) lors d'un déplacement du corps de seringue (3) par rapport à l'élément manchon (6),
dans lequel, lors d'une utilisation de la seringue (2), ladite au moins une saillie de guidage (13) est guidée d'une première zone de coulisse (18) à une seconde zone de coulisse (19) et une partie du moyen de piqûre (5) passe à travers la face d'ouverture (9) et dans un angle différent de 90° dans la peau du patient.

2. Corps de seringue (3) selon la revendication 1, dans lequel l'angle α se trouve à 45°.

3. Corps de seringue (3) selon la revendication 1, dans lequel l'élément collet (11) est essentiellement formé en tant que cylindre creux à base circulaire (16), le cylindre à base circulaire (16) présentant une surface d'enveloppe (16a) sur laquelle ladite au moins une saillie de guidage (13) est disposée.

4. Corps de seringue (3) selon l'une des revendications précédentes, dans lequel la partie de contact (8) comporte une face frontale (15), laquelle présente une structure de surface rugueuse et/ou est faite d'une matière caoutchoutée.

5. Corps de seringue (3) selon l'une des revendications précédentes, dans lequel la face d'ouverture (9) présente une autre zone partielle (17), laquelle forme un angle β avec la direction axiale (X), l'angle β étant compris dans une plage entre 80° et 100°.

6. Corps de seringue (3) selon l'une des revendications précédentes, dans lequel la coulisse de guidage (14) comporte une première (18) et une seconde zone de coulisse (19) qui sont séparées l'une de l'autre par une ligne de séparation fictive (20) s'étendant le long de la direction axiale (X) du corps de seringue (3), la saillie de guidage (13) étant apte à être disposée dans une position initiale dans la première zone de coulisse (18).

7. Corps de seringue (3) selon la revendication 6, dans lequel, dans la position initiale, une extrémité distale (21) du moyen de piqûre (5) est disposée dans la direction axiale (X) à l'intérieur de l'élément manchon, la saillie de guidage (13) étant transférable de la première (18) dans la seconde zone de coulisse (19) dans une position finale en franchissant la ligne de séparation (20) lorsqu'une extrémité distale (21) du moyen de piqûre (5) est disposée dans la face d'ouverture (10) lors du déplacement relatif du corps de seringue (3) par rapport à l'élément manchon (6).

8. Corps de seringue (3) selon la revendication 6, dans lequel, dans la position initiale, une extrémité distale (21) du moyen de piqûre (5) est disposée dans la face d'ouverture (9).

9. Corps de seringue (3) selon la revendication 6, dans lequel, dans la position initiale, une extrémité distale (21) du moyen de piqûre (5) fait saillie au-delà de l'élément manchon dans la direction axiale (X).

10. Corps de seringue (3) selon l'une des revendications précédentes, dans lequel le dispositif de sécurité (1) présente au moins un élément ressort, qui est relié fonctionnellement avec le corps de seringue (3) et s'oppose au déplacement relatif du corps de seringue (3) par rapport à l'élément manchon (6).

11. Corps de seringue (3) selon l'une des revendications précédentes, dans lequel ladite au moins une saillie de guidage (13) est transférable de la seconde zone de coulisse (20) au moyen d'une coulisse de la seconde zone de coulisse (19) dans une zone d'extrémité (24), dans laquelle un déplacement relatif de l'élément manchon (6) par rapport au corps de seringue (3) est au moins limité sensiblement le long de la direction axiale (X).

12. Corps de seringue (3) selon l'une des revendications précédentes, dans lequel le dispositif de sécurité (1) comporte un élément capuchon, lequel est disposé sur l'extrémité distale (7) de l'élément manchon (6).

13. Corps de seringue (3) selon l'une des revendications précédentes, dans lequel le moyen de piqûre (5) comporte un tranchant et une pointe, le moyen de piqûre (5) étant disposé de telle sorte que sa pointe est disposée dans une direction s'opposant diamétralement à la hauteur maximale de l'élément manchon (6).
